# EUROPÄISCHE PATENTSCHRIFT

(11) **EP 1 474 180 B1**
(45) Veröffentlichungstag und Bekanntmachung des Hinweises auf die Patenterteilung: **27.07.2005**
(21) Anmeldenummer: 03706398.9
(22) Anmeldetag: 24.01.2003
(51) Int. Cl.: A61L 2/28

(54) **MESSEINRICHTUNG ZUM ÜBERWACHEN VON STERILISATIONSBEDINGUNGEN**
MEASURING DEVICE FOR MONITORING STERILIZATION CONDITIONS
DISPOSITIF DE MESURE POUR CONTROLER DES CONDITIONS DE STERILISATION

(30) Priorität: 05.02.2002 DE 20201752 U
(43) Veröffentlichungstag der Anmeldung: 10.11.2004
(73) Patentinhaber: HS System- und Prozesstechnik Gmbh, 65779 Kelkheim (DE)
(72) Erfinder: HÜCKER, Gerhard, 65779 Kelkheim (DE)
(74) Vertreter: Meyer-Dulheuer, Karl-Hermann, Dr.
(86) Internationale Anmeldenummer: PCT/EP2003/000895
(87) Internationale Veröffentlichungsnummer: WO 2003/066111

(56) Entgegenhaltungen:
- EP-A- 0 808 631
- EP-A- 1 025 863
- EP-A- 1 172 117
- WO-A-01/07092
- US-A- 5 422 276

## Beschreibung

Gegenstand der Erfindung ist eine Messeinrichtung zum Überwachen der Sterilisationsbedingungen in einer Sterilisadonskammer, bestehend aus einem Datenaufnehmer, der in die Sterilisationskammer eingebracht werden kann, einem Datenauslesegerät außerhalb der Sterilisationskammer und einer Auswerteeinheit.

Messeinrichtungen zum Überwachen der Sterilisationsbedingungen in einer Sterilisationskammer sind bereits bekannt. Die europäische Patentanmeldung EP A 1 172 117 beschreibt eine Testvorrichtung, die eine Vielzahl von Temperatursensoren enthält. Die Testvorrichtung wird in der Sterilisationskammer eingesetzt, so dass die Temperaturen an einer Vielzahl von Stellen in der Sterilisationskammer gemessen werden können. Mit den dort beschriebenen Temperatursensoren wird die Kammertemperatur an vorbestimmten festen Punkten gemessen, um einen Vergleichswert für die Temperaturen in einer "process-challenge-device" zu erhalten. Die internationale Patentanmeldung WO 93/21964 sowie die Anmeldung US-A-5 422 276 beschreiben eine Vorrichtung zur Überwachung der Sterilisation, bei der die innerhalb der Sterilisationskammer ermittelten Messbedingungen durch Funkübertragung an einen externen Datenschreiber übertragen werden. Die internationale Patentanmeldung WO 95/32742 beschreibt zudem eine Testvorrichtung zum Ermitteln der Sterilisationsbedingungen in einem Sterilisationsapparat. Hierbei werden an zwei genau vorbestimmten Stellen innerhalb des Sterilisationsapparates die Sterilisationsbedingungen genau gemessen.

Derartige Messeinrichtungen bestehen stets aus zwei Teilen, und zwar dem Messgerät, das die Sterilisationsbedingungen der Sterilisationskammer misst, und einem zweiten Teil, dem Datenauswertegerät. Das Messgerät bleibt hierbei so lange in der Sterilisationskammer, bis der Sterilisationsvorgang vollständig abgeschlossen ist. In vorgegebenen Zeitintervallen werden von diesem Messgerät die Parameter Druck, Temperatur und Feuchtigkeit gemessen und entweder über eine Antenne auf das Auswertegerät übertragen oder die ermittelten Daten werden abgespeichert und nach Entnahme des Messgerätes aus der Sterilisationskammer ausgewertet. Somit ist es möglich zu dokumentieren, dass die in der Steritisationskammer behandelten Gegenstände korrekt und vorschriftsmäßig sterilisiert wurden. Die Temperaturmessung erfolgt in den beiden vorstehend genannten Ausführungsformen ausschließlich an einem oder an zwei Punkten innerhalb der Sterilisationskammer.

Es hat sich jedoch in einer Vielzahl von Untersuchungen herausgestellt, dass lokale Temperaturgradienten oder lokale Temperaturunterschiede einen erheblichen Einfluss auf die Güte der Sterilisation haben. Um sicherzustellen, dass die Sterilisationstemperatur in der gesamten Sterilisationskammer ausreichend hoch ist, ist es notwendig, die Temperatur an mehreren Stellen in der Sterilisationskammer zu messen. Die bisher eingesetzten Temperatursensoren gestatteten es jedoch nicht, mehrere Temperatursensoren in einem einzigen Messgerät zu kombinieren und zudem ein kurzes thermisches Ansprechverhalten zu gewährleisten.

Gelöst wird diese Aufgabe durch eine Messeinrichtung zum Überwachen der Sterilisationsbedingungen in einer Sterilisationskammer, bestehend aus einem Datenaufnehmer 1, der in die Sterilisationskammer eingebracht wird Temperatursensoren 6, einem Datenspeicher 10 zum Speichern der gemessenen Temperaturen, einem Datenauslesegerät 2 außerhalb der Sterilisationskammer und einer Auswerteeinheit 3, wobei der Datenaufnehmer 1 ein Gehäuse 7 aufweist, in dem sich ein Steuergerät 8, eine Stromversorgungseinheit 9, ein Datenspeicher 10 und ein Drucksensor 13 befinden und das Datenauslesegerät 2 mit einem Datenausgang 12 versehen sind, wobei die in die Sterilisationskammer eingeführten, aus Halbleiterelementen bestehenden Temperatursensoren 6 über flexible Sensorkabel 5 mit dem Datenaufnehmer 1 verbunden sind, so dass an jeder Stelle der Sterilisationskammer die genaue Temperatur messbar ist.

Durch die erfindungsgemäße Ausgestaltung der Temperatursensoren als Halbleiterelemente besteht die Möglichkeit, die Temperatur an verschiedenen Stellen der Sterilisationskammer mit einem kurzen thermischen Ansprechverhalten genau zu bestimmen. Die erfindungsgemäße Messeinrichtung wird anhand der beiliegenden Zeichnungen näher erläutert.

Es zeigen:
- Fig. 1: die schematische Darstellung der erfindungsgemäßen Messeinrichtung zum Überwachen der Sterilisationsbedingungen und
- Fig. 2: eine schematische Darstellung des Datenaufnehmers.

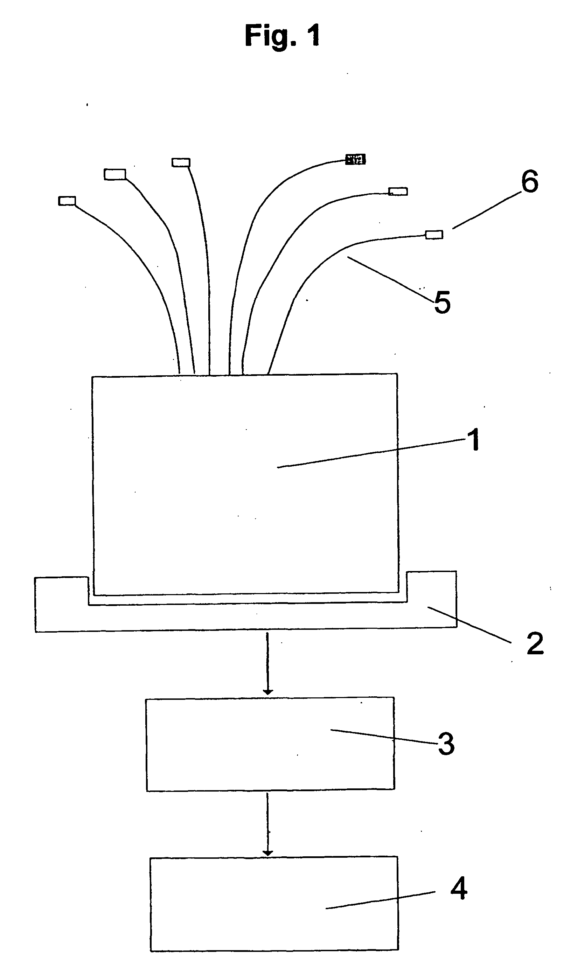

Fig. 1 zeigt die erfindungsgemäße Messeinrichtung, bestehend aus dem Datenaufnehmer 1, der über Sensorkabel 5 mit in die Sterilisationskammer einführbaren Halbleiterelementen 6 verbunden ist, durch die an einer Vielzahl von Stellen in der Sterilisationskammer Temperaturen gemessen werden können. Diese Temperaturen werden im Datenaufnehmer gespeichert und können von dem Datenauslesegerät 2 ausgelesen werden. Sie können dann von dort über eine Auswerteeinheit z. B. einen Personal Computer, optisch und graphisch sichtbar gemacht werden und mit einem angeschlossenen Drucker 4 ausgedruckt werden.

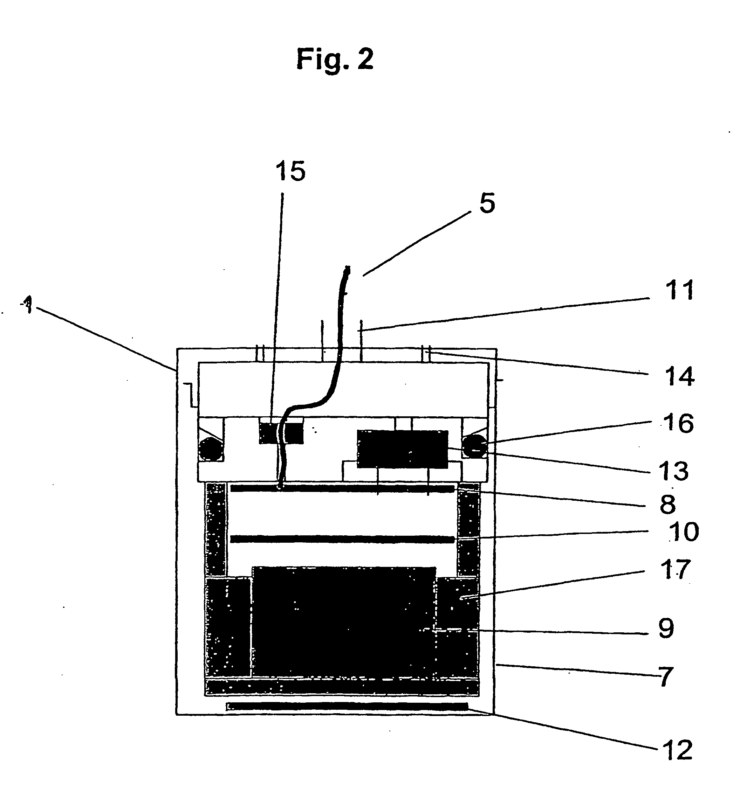

Fig. 2 zeigt den Datenaufnehmer, der sich zur Überwachung des Sterilisationsprozesses besonders eignet. Der erfindungsgemäße Datenaufnehmer besteht aus einem äußeren Gehäuse 7, das aus Edelstahl, Kunststoff oder einem anderen geeigneten druck- und temperaturbeständigen sowie innerten Material hergestellt sein kann. Im Inneren des Gehäuses 7 befinden sich,ein Steuergerät 8, eine Stromversorgungseinheit 9, ein Datenspeicher 10 und ein Drucksensor 13. Bei der Stromversorgungseinheit 9 kann es sich beispielsweise um einen Akku handeln, der über Ladekontakte, die nicht gezeigt werden, aufgeladen wird. Die Energieversorgung kann jedoch auch über eine Batterie erfolgen, die in einem dafür vorgesehenen Batteriefach eingelegt ist. Das Batteriefach ist von einer thermischen Isolation 17 umgeben, um eine Schädigung bei den hohen Bedingungen in er Sterilisationskammer zu verhindern. Außerdem sind ein Drucksensor 13, ein Druckausgleich 14 sowie ein hier noch vorgesehener Sensor zur Messung der Luftfeuchtigkeit 15 zu erkennen. In der dargestellten Ausführungsform ist der Drucksensor 13 direkt an der Innenseite des Gehäuses 7 untergebracht. Das Steuergerät 8 ist mit den einzelnen Sensoren verdrahtet. Die von den Sensoren gemessenen Daten werden dem Steuergerät 8 zugeführt, dort verarbeitet und anschließend im Datenspeicher 10 abgespeichert.

Der Datenspeicher 10 ist mit dem Datenausgang 12 verbunden, wobei es sich bei dem Datenübergang um eine Kontaktplatte zur elektrischen Datenübertragung handeln kann. Es ist jedoch auch möglich, durch optische, magnetische oder akustische Datenübertragung die Daten an das Datenauslesegerät zu übertragen. Die aus Halbleiterelementen bestehenden Temperatursensoren 6 sind mit flexiblem Sensorkabeln 5 verbunden und können so an jeder Stelle des Sterilisationsraumes die genaue Temperatur messen. Die verwendeten Halbleiterelemente zeichnen sich durch ein kurzes thermisches Ansprechverhalten wie auch eine entsprechend geringe Dimensionierung aus. In einer besonders bevorzugten Ausführungsform können 8 oder sogar 16 Thermosensoren mit dem Steuergerät 8 verbunden sein. Somit ist eine genaue Temperaturbestimmung an allen Stellen innerhalb des Sterilisationsraumes möglich. Das Gehäuse 7 weist eine Öffnung 11 zur Kabeldurchführung zur Sterilisationskammer auf. Außerdem ist ein Dichtring 16 vorgesehen, der das Eindringen der heißen, feuchten und unter Druck stehenden Luft in den Datenlogger verhindert.

Das in Fig. 1 schematisch gezeichnete Auslesegerät 2 ist zweckmäßigerweise mit einem Auswertegerät, z. B. einem Personal Computer 3 und einem Drucker 4 verbunden. Das Datenauslesegerät 2 ist vorteilhafterweise mit einer Vertiefung versehen, in die der Datenaufnehmer 1 eingesetzt werden kann, so dass eine Datenübertragung zwischen dem Datenaufnehmer 1 und dem Datenauslesegerät 2 gewährleistet ist. Dabei wird der Datenlogger über zwei Federkontakte kontaktiert. Der Datenaustausch kann über eine sogenannte Zweidrahtschnittstelle erfolgen. Das Auslesegerät kann zweckmäßigerweise über den USB-Bus mit Spannung versorgt werden. Ein zusätzliches Netzteil kann entfallen.

Die besonderen Vorteile der erfindungsgemäßen Messeinrichtung werden durch den Einsatz von Halbleiterelementen als Temperaturfühler erreicht. Sie weisen kurze thermische Ansprechzeiten auf, haben eine hohe Langzeitstabilität und erlauben durch den Einsatz von bis zu 16 Temperaturfühlern eine exakte Darstellung der Temperaturverhältnisse in der Sterilisationskammer. Hinzu kommt, dass die Auswerteelektronik gut beherrschbar ist, während die Auswertung des Messsignals von den bisher für derartige Einsatzzwecke verwendeten Thermoelementen in einem kleinen Logger im Sterilisator nicht realisierbar ist.

### Bezugszeichenliste:

- 1: Datenaufnehmer (Datenlogger)
- 2: Datenauslesegerät
- 3: Auswerteeinheit (PC)
- 4: Drucker
- 5: Sensorkabel
- 6: Halbleiterelement (Temperatursensor)
- 7: Gehäuse
- 8: Steuergerät
- 9: Stromversorgungseinheit
- 10: Datenspeicher
- 11: Kabeldurchführung zur Sterilisationskammer
- 12: Kontaktplatte zur Datenübertragung
- 13: Drucksensor
- 14: Druckausgleich
- 15: Sensor zur Messung der Luftfeuchtigkeit
- 16: Dichtring
- 17: Thermische Isolation

## Patentansprüche

1. Messeinrichtung zum Überwachen der Sterilisationsbedingungen in einer Steritisationskammer bestehend aus einem Datenaufnehmer (1), der in die Sterilisationskammer eingebracht wird, Temperatursensoren (6), einem Datenspeicher (10) zum Speichern der gemessenen Temperaturen, einem Datenauslesegerät (2) außerhalb der Sterilisationskammer und einer Auswerteeinheit (3), wobei der Datenaufnehmer (1) ein Gehäuse (7) aufweist, in dem sich ein Steuergerät (8), eine Stromversorgungseinheit (9) und ein Drucksensor (13) befinden und das Datenauslesegerät (2) mit einem Datenausgang (12) versehen ist, **dadurch charakterisiert,** dass die in die Sterilisationskammer eingeführten, aus Halbleiterelementen bestehenden Temperatursensoren (6) über flexible Sensorkabel (5) mit dem Datenaufnehmer (1) verbunden sind, so dass an jeder Stelle der Sterilisationskammer die genaue Temperatur messbar ist.

2. Messeinrichtung gemäß Anspruch 1, **dadurch charakterisiert,** dass der Drucksensor (13) im Gehäuse (7) des Datenaufnehmers (1) untergebracht ist.

3. Messeinrichtung gemäß den Ansprüchen 1 und 2, **dadurch charakterisiert,** dass der Datenaufnehmer (1) neben einem Drucksensor (13) und mehreren Temperatursensoren (6) einen weiteren Sensor (15) zum Messen der relativen Luftfeuchtigkeit aufweist.

4. Messeinrichtung gemäß den Ansprüchen 1 bis 3, **dadurch charakterisiert,** dass die Datenübertragung vom Datenaufnehmer (1) zum Datenauslesegerät (2) elektrisch, optisch, magnetisch oder akustisch erfolgt.

## Claims

1. Measuring device for monitoring the sterilisation conditions in a sterilisation chamber comprising a data recording device (1), which is incorporated in the sterilisation chamber, temperature sensors (6), a data storage device (10) for storing the measured temperatures, a data read-out device (2) outside the sterilisation chamber and an evaluation unit (3), wherein the data recording device (1) has a housing (7) containing a controller (8), a power supply unit (9) and a pressure sensor (13) and the data read-out device (2) is provided with a data output (12) **characterised in that** the temperature sensors (6) comprising semiconductor elements, which are inserted in the sterilisation chamber, are connected to the data recording device (1) via flexible sensor cable (5) so that the exact temperature can be measured at every point of the sterilisation chamber.

2. The measuring device according to claim 1, **characterised in that** the pressure sensor (13) is accommodated in the housing (7) of the data recording device (1).

3. The measuring device according to claims 1 and 2, **characterised in that** in addition to a pressure sensor (13) and a plurality of temperature sensors (6), the data recording device (1) has a further sensor (15) for measuring the relative air humidity.

4. The measuring device according to claims 1 to 3, **characterised in that** the data transmission from the data recording device (1) to the data read-out device (2) takes place electrically, optically, magnetically or acoustically.

## Revendications

1. Dispositif de mesure pour le contrôle des conditions de stérilisation dans une chambre de stérilisation, composé d'un enregistreur de données (1) qui est intégré dans la chambre de stérilisation, de capteurs de température (6), d'une mémoire de données (10) pour le stockage des températures mesurées, d'un appareil de lecture de données (2) extérieur à la chambre de stérilisation et d'une unité d'exploitation (3), l'enregistreur de données (1) présentant un boîtier (7) dans lequel se trouvent un appareil de commande (8), une unité d'alimentation en courant (9) et un capteur de pression (13) et l'appareil de lecture de données (2) étant équipé d'une sortie dé données (12), **caractérisé en ce que** les capteurs de température (6) introduits dans la chambre de stérilisation et consistant en éléments semi-conducteurs sont reliés par des câbles de détecteurs flexibles (5) à l'enregistreur de données (1), de sorte que la température exacte peut être mesurée à tout endroit de la chambre de stérilisation.

2. Dispositif de mesure selon la revendication 1, **caractérisé en ce que** le capteur de pression (13) est logé dans le boîtier (7) de l'enregistreur de données (1).

3. Dispositif de mesure selon les revendications 1 et 2, **caractérisé en ce que** l'enregistreur de données (1), outre un capteur de pression (13) et plusieurs capteurs de température (6), présente un autre capteur (15) pour mesurer l'humidité relative de l'air.

4. Dispositif de mesure selon les revendications 1 et 3, **caractérisé en ce que** la transmission des données de l'enregistreur de données (1) à l'appareil de lecture de données (2) a lieu de manière électrique, optique, magnétique ou acoustique.
